Europäisches Patentamt

European Patent Office

Office européen des brevets

(11) Numéro de publication : **0 008 264**
**B1**

# FASCICULE DE BREVET EUROPÉEN

(45) Date de publication du fascicule du brevet :
09.06.82

(21) Numéro de dépôt : 79400521.5

(22) Date de dépôt : 20.07.79

(51) Int. Cl.³ : **A 61 B  6/02**, A 61 B  6/06,
G 21 K  1/10, G 05 D  3/00

(54) **Procédé de centrage automatique d'un objet à examiner dans un tomodensitomètre à faisceau en éventail, et tomodensitomètre adapté à ce procédé.**

(30) Priorité : 27.07.78 FR 7822268

(43) Date de publication de la demande :
20.02.80 (Bulletin 80/04)

(45) Mention de la délivrance du brevet :
09.06.82 Bulletin 82/23

(84) Etats contractants désignés :
DE GB NL SE

(56) Documents cités :
DE A 2 452 166
FR A 2 260 977
FR A 2 329 132
FR A 2 344 032
FR A 2 345 983
FR A 2 406 208

(73) Titulaire : COMPAGNIE GENERALE DE RADIOLOGIE
13 square Max-Hymans
F-75741 PARIS CEDEX 15 (FR)

(72) Inventeur : Klausz, Rémy
"THOMSON-CSF" - SCPI 173, bld Haussmann
F-75360 Paris Cedex 08 (FR)

(74) Mandataire : Thierr, Françoise et al
THOMSON-CSF SCPI 173, Bld Haussmann
F-75360 Paris Cedex 08 (FR)

Imprimerie Jouve, 18, rue St-Denis, 75001 Paris, France

# Procédé de centrage automatique d'un objet à examiner dans un tomodensitomètre à faisceau en éventail, et tomodensitomètre adapté à ce procédé

L'invention concerne un procédé de centrage automatique d'un objet à examiner dans un tomodensitomètre à faisceau en éventail, et un tomodensitomètre adapté à ce procédé.

Un tel appareil se compose essentiellement d'une source émettant un faisceau de rayons en forme d'éventail en direction de plusieurs détecteurs disposés en ligne sur un arc de cercle et définissant un secteur du plan de coupe à l'intérieur duquel se trouve l'objet à examiner, source et détecteurs étant montés sur un support mobile en rotation autour d'un axe normal au plan de coupe, et situé sensiblement au centre de l'objet à examiner. Un ordinateur reçoit les signaux émis par chaque détecteur à chaque station du support mobile au cours de sa rotation. Il les traite et calcule pour chaque point de la coupe, la quantité de rayons ayant traversé chacun des points correspondants du corps à examiner, au cours des différentes stations.

Pour que ce calcul soit exact il est indispensable que la totalité des points de l'objet à examiner soit, à chaque station, située à l'intérieur du secteur déterminé par la source et les détecteurs, autrement dit qu'ils soient situés à l'intérieur de la zone appelée champ de mesure, qui est un cercle défini comme l'enveloppe des rayons venant frapper les détecteurs d'extrémité de la ligne des détecteurs au cours de la rotation du support mobile pendant un cycle de mesure. S'il n'en était pas ainsi, l'image que l'on obtiendrait serait une représentation erronée. Il est donc nécessaire d'effectuer un centrage correct du corps à examiner.

On peut pour cela utiliser des dispositifs simulant le champ de mesure, dispositifs soit mécaniques, par exemple une ouverture circulaire, soit visuels, par exemple une zone ou des traits lumineux. Ceci suppose que ces dispositifs soient eux-mêmes centrés avec précision sur le champ de mesure réel, ce qui demande des opérations préalables très longues et un très grand soin. De plus l'utilisation de tels systèmes est loin d'être simple et rapide.

Un autre moyen de réaliser un centrage précis et rapide consiste à associer au support mobile un dispositif capteur de position, tel qu'une association de faisceaux lumineux avec des capteurs optiques. Mais il faut alors ajouter à l'appareil des circuits électroniques supplémentaires reliant ce dispositif, soit à un dispositif d'exploitation directe par l'utilisateur, soit à l'ordinateur.

Par ailleurs le procédé consistant à effectuer un cycle complet de mesures destiné à vérifier le bon centrage du corps à examiner (et à le rectifier éventuellement) doit être exclu principalement en raison de l'importance de la dose de rayonnement qu'il infligerait au corps à examiner.

Le procédé selon l'invention évite les inconvénients signalés. Il est basé sur le fait que la position de bon centrage est réalisée, si quelle que soit la position du support mobile, la mesure la plus extérieure est faite hors de l'objet à examiner. Les détecteurs sont alors sensibles à l'absence d'objet sur le trajet des rayons qui les frappe, et envoient au calculateur le signal caractéristique correspondant.

Ce procédé est caractérisé essentiellement en ce qu'il consiste :

— à placer entre la source et l'objet à examiner un écran opaque aux rayons, mobile entre une position centrage et une position effacée, de dimensions telles qu'en position centrage il occulte les rayons frappant les détecteurs sauf au moins un détecteur à au moins une des extrémités de la ligne des détecteurs ;

— à programmer l'ordinateur pour obtenir, en réponse aux signaux qu'il recevra du ou des détecteurs non occultés par l'écran, au cours d'un cycle de mesures, l'indication de la direction et de la grandeur du déplacement à faire effectuer au corps à examiner pour qu'aucune partie dudit corps ne soit placée, au cours d'un cycle de mesure, entre la source et les détecteurs non occultés situés aux extrémités de la ligne des détecteurs ;

— et à effectuer un cycle de mesures avec l'appareil ainsi disposé en position centrage.

Il peut être appliqué à différents types d'appareils (tomodensitomètres à translation et rotation ou rotation seule). Cependant il est particulièrement simple à appliquer à un tel appareil à faisceau en éventail qui est à rotation seule.

Aucun document de l'état antérieur de la technique ne suggère une telle utilisation d'un écran amovible utilisé au cours d'une opération de centrage préalable au cycle de mesures proprement dite d'un tomodensitomètre à faisceau en éventail.

En effet, le seul document antérieur concernant un tomodensitomètre à rayons X utilisant un obturateur de faisceau en liaison avec la position du sujet à examiner est la demande de brevet français FR-A-2.260.977. Mais il apparaît clairement que les ressemblances avec la présente invention s'arrêtent là puisqu'en effet ce document concerne un tomodensitomètre à un seul détecteur dans lequel l'obturateur a pour but (page 2 lignes 31 et 32) d'éviter l'utilisation d'un sac d'eau pour effectuer les examens. Ceci n'a bien entendu rien à voir avec un centrage préalable, le sac d'eau (ou ici l'obturateur qui le remplace) étant uniquement destiné à éviter les trop fortes transitions lors du passage du faisceau d'examen du sujet à examiner à l'air qui l'aurait entouré en l'absence de ce sac d'eau (ou de l'obturateur).

Ainsi donc un obturateur avait déjà été utilisé pour l'examen du sujet, mais pas pour une opération de centrage préalable à l'examen proprement dit.

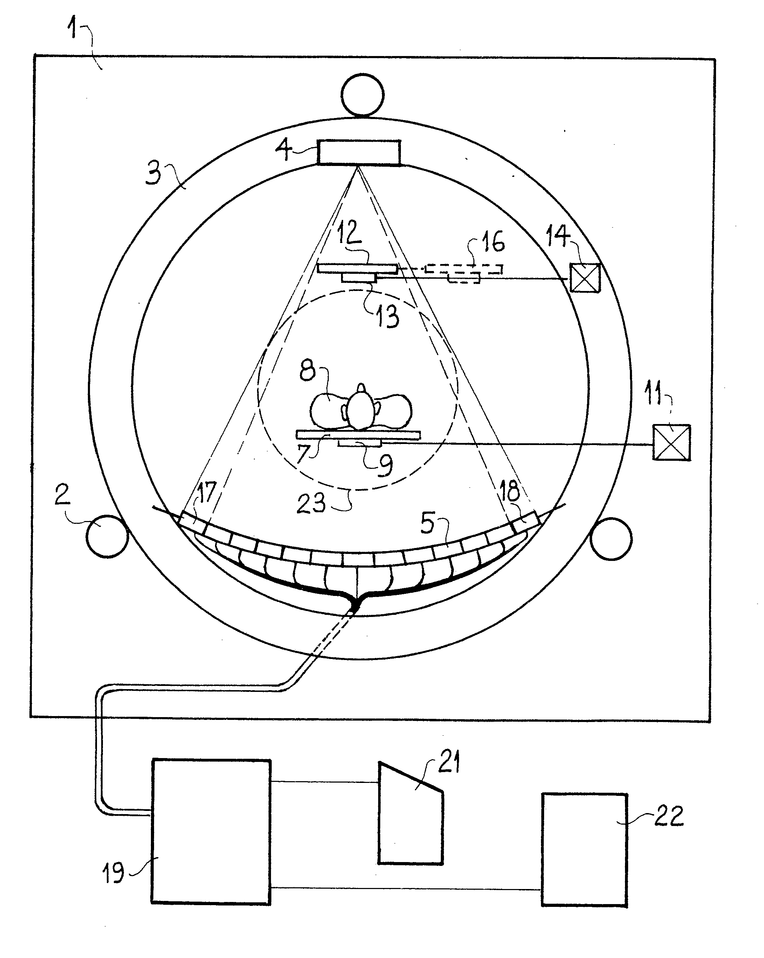

D'autres caractéristiques de l'invention pourront apparaître au cours de la description d'une application de ce procédé à un tomodensitomètre à faisceau en éventail, donnée ci-après avec l'aide de la figure unique qui représente ledit tomodensitomètre. On voit le bâti 1 supportant un dispositif de rotation composé de trois galets 2 entre lesquels roule le support mobile 3 sur lequel sont fixés une source 4 et une ligne de détecteurs 5. Le bâti 1 est évidé entre les galets 2 de manière à pouvoir laisser pénétrer à l'intérieur du support 3 un support 7 pour le corps à examiner 8. Ce support 7 est fixé sur un dispositif 9 lui-même fixé sur le bâti 1 et susceptible de se déplacer sous l'effet d'une commande 11 dans une direction quelconque dans le plan de la figure.

Selon une caractéristique principale de l'invention un écran 12 opaque aux rayons est placé entre la source 4 et le corps à examiner 8. Cet écran est fixé sur un dispositif 13 solidaire du support 3, qui sous l'effet d'une commande 14 permet de déplacer ledit écran 12 entre une position de centrage indiquée en 12 et une position effacée 16 indiquée en pointillé. Les dimensions de l'écran 12 sont telles qu'en position de centrage, il empêche les rayons de venir frapper les détecteurs de la ligne 5 sauf au moins un des détecteurs 17 ou 18 aux extrémités de la ligne 5.

Les détecteurs de la ligne 5 sont reliés à un ordinateur 19 recevant des instructions, et notamment des programmes du pupitre de commande 21, et qui envoie le résultat de ses calculs à un dispositif d'exploitation des résultats 22.

Le tomodensitomètre à centrage automatique fonctionne de la manière suivante. Au cours des stations de mesures décrites par le support 3 dans sa rotation, les rayons venant frapper les détecteurs d'extrémité 17, 18 délimitent le champ de mesures représenté sur la figure par le cercle en pointillé 23 à l'intérieur duquel doit se trouver entièrement le corps à examiner 8 sous peine d'obtenir une image erronée. Cette condition sera remplie si, au cours d'un cycle de mesures, aucun des détecteurs 17 ou 18 n'est occulté par une partie du corps à examiner. Une telle occultation provoque une absorption du rayonnement à laquelle les détecteurs sont sensibles. Ces derniers envoient à l'ordinateur 19 les signaux correspondants. Si aucun des détecteurs 17 ou 18 n'est occulté, l'ordinateur ne donne aucune indication autre qu'une position correcte du corps à examiner 8. Si un de ces deux détecteurs est occulté au cours du cycle de mesures, l'ordinateur 19, dûment programmé par le pupitre 21, élabore en fonction des positions du support 3 pour lesquelles ont eu lieu les occultations, la direction et la grandeur du déplacement à imposer au support 7 pour que le corps à examiner 8 ne vienne plus occulter les détecteurs 17 ou 18.

Pour éviter une dose d'irradiation trop forte au corps à examiner, on déplace par le dispositif 14 l'écran 12 de la position effacée 16 à la position centrage de manière que tous les rayons émis par la source 4 soient interceptés par l'écran, sauf ceux qui vont frapper les détecteurs d'extrémité 17 et 18.

En résumé la mise en position centrage du tomodensitomètre provoque :

— la mise en position centrage de l'écran 12 ;
— l'envoi à l'ordinateur du programme de centrage ;
— le déclenchement d'un cycle de mesures (cycle de centrage).

Les indications concernant le centrage sont soit recueillies par le dispositif 22, soit envoyés directement au dispositif 11 qui commande automatiquement les déplacements convenables du support 7 du corps à examiner 8.

On voit que les détecteurs étant disposés symétriquement par rapport à l'axe de rotation du support 3, le cercle 23 délimitant le champ de mesure est entièrement enveloppé par le rayonnement reçu par un seul des détecteurs 17 ou 18 effectuant un tour complet de rotation. Ainsi, dans une variante, l'écran occulte la totalité des détecteurs sauf un seul situé à une extrémité de la ligne 5.

Certains densitomètres sont équipés pour disposer d'un champ de mesure de grandeur variable. Cette variation est obtenue soit en diminuant l'ouverture du faisceau en éventail émis par la source 4, soit en faisant varier la position de la source par rapport au centre de rotation du support 3. Dans un cas comme dans l'autre l'angle d'occultation de l'écran 12 doit être adapté pour que seuls le ou les détecteurs d'extrémité de la ligne de détecteurs 5 ne soient pas occultés. Ceci peut être réalisé de deux manières différentes. Le dispositif 13 est susceptible de recevoir des écrans 12 de plusieurs dimensions : on choisit alors l'écran dont la dimension permet d'occulter les détecteurs sauf le ou les détecteurs d'extrémité pour le champ de mesure adopté. Une autre manière consiste à donner au dispositif 13 la possibilité de se déplacer vers la source 4 (ou en sens contraire) sous l'action de la commande 14. On donne alors à l'écran 12 la position voulue en fonction de la dimension du champ de mesure.

## Revendications

1. Procédé de centrage automatique d'un objet (8) à examiner dans un tomodensitomètre à faisceau en éventail, le tomodensitomètre comportant essentiellement une source (4) émettant un faisceau de rayons en forme d'éventail en direction de plusieurs détecteurs (5) disposés en ligne et définissant un secteur du plan de coupe à l'intérieur duquel se trouve un support (7) de l'objet à examiner, source et détecteurs étant montés sur un support (3) mobile en rotation autour d'un axe normal au plan de coupe au moyen d'un dispositif de rotation (2) fixé sur un bâti (1), ledit support (7) de l'objet à examiner étant fixé au bâti par un dispositif (9) permettant de lui faire effectuer une translation dans une direction quelconque, ce tomodensitomètre comportant en outre, un ordinateur (19) recevant les signaux émis par chaque détecteur (5) à chaque station dudit support mobile (3) au cours de sa rotation, un pupitre de commande (21) notamment de l'ordinateur (19) et un dispositif d'exploitation (22) des résultats émis par l'ordinateur (19) en fonction des signaux reçus des détecteurs (5), et du programme reçu du pupitre de commande (21), procédé caractérisé en ce qu'il consiste :

— à placer entre la source (4) et l'objet (8) à examiner un écran (12) opaque aux rayons, mobile entre une position centrage (12) et une position effacée (16), de dimensions telles qu'il occulte en position centrage les rayons frappant les détecteurs (5) sauf au moins un détecteur (17, 18) à au moins une des extrémités de la ligne des détecteurs (5),

— à envoyer à l'ordinateur (19) un programme pour obtenir, en réponse aux signaux qu'il recevra du ou des détecteurs (17, 18) non occultés par l'écran (12), au cours d'un cycle de mesures, l'indication de la direction et de la grandeur du déplacement à faire effectuer au support (7) du corps (8) à examiner pour qu'aucune partie dudit corps ne soit placée au cours d'un cycle de mesures, entre la source (4) et les détecteurs (17, 18) non occultés par l'écran (12) situés aux extrémités de la ligne des détecteurs (5),

— et à effectuer un cycle de mesures avec l'appareil ainsi disposé en position centrage.

2. Tomodensitomètre à faisceau en éventail comportant essentiellement une source (4) émettant un faisceau de rayons en forme d'éventail en direction de plusieurs détecteurs (5) disposés en ligne et définissant un secteur du plan de coupe à l'intérieur duquel se trouve un support (7) de l'objet (8) à examiner, source (4) et détecteurs (5) étant montés sur un support (3) mobile en rotation autour d'un axe normal au plan de coupe au moyen d'un dispositif de rotation (2) fixé sur un bâti (1), le support (7) de l'objet (8) à examiner étant fixé au bâti (1), ce tomodensitomètre comportant en outre un ordinateur (19) recevant les signaux émis par chaque détecteur (5) à chaque station du support mobile (3) au cours de sa rotation, un pupitre de commande (21) notamment de l'ordinateur (19), et un dispositif d'exploitation (22) des résultats émis par l'ordinateur (19), en fonction des signaux reçus des détecteurs (5), et du programme reçu du pupitre de commande (22), tomodensitomètre caractérisé en ce qu'il comporte en outre un écran (12) opaque aux rayons, mobile entre une position centrage (12) et une position effacée (16), situé en position centrage entre la source (4) et l'objet (8) à examiner, cet écran (12) ayant des dimensions telles qu'il occulte en position centrage (12) les rayons frappant les détecteurs sauf au moins un détecteur (17, 18) situé à au moins une des extrémités de la ligne des détecteurs (5).

3. Tomodensitomètre selon la revendication 2, caractérisé en ce que l'écran opaque (12) est fixé dans un dispositif (13) apte à recevoir des écrans opaques de différentes dimensions.

4. Tomodensitomètre selon la revendication 2, caractérisé en ce que l'écran opaque (12), est fixé sur un dispositif (13) susceptible de se déplacer en direction de la source (4) ou en sens contraire sous l'action (13) d'une commande.

5. Tomodensitomètre selon la revendication 2, caractérisé en ce que le support (7) de l'objet (8) à examiner est fixé au bâti (1) par l'intermédiaire d'un dispositif (9) susceptible de lui imprimer sous l'action d'une commande un déplacement dans une direction quelconque.

## Claims

1. A method of automatically centring an object (8) to be examined within a fan-beam tomodensitometer, the tomodensitometer consisting substantially of the following components : a source (4) emitting a fan-shaped beam of rays towards a plurality of detectors (5) arrayed in one line and defining a sector of the cutting plane in the interior of which there is provided a support (7) for the object to be examined, in which the source and the detectors are mounted on a support (3) rotatable by a rotating means (2) attached to a frame (1) about an axis perpendicular to the cutting plane, and the support (7) for the object to be examined is attached to the frame by a means (9) permitting the support to be moved in any desired direction ; a computer (19) which receives the signals emitted by each of the detectors (5) in any position of the rotatable support (3) during rotation thereof, a control console (21) mainly for controlling the computer (19) and a device (22) for evaluating the results outputted by the computer (19) as a function of the signals received from the detectors (5) and the programme received from the control console (21) ; the method being characterised by

— providing between the source (4) and the object (8) to be examined a screen (12) which is opaque and movable between a centred position (12) and a retracted position (16) and which is dimensioned such that in the centred position thereof it will keep off the rays striking the detectors (5) except for at least one detector (17, 18) at at least one end of the line of detectors (5) ;

— entering a programme into the computer (19) in order to obtain in response to the signals which the computer receives from the detector or detectors (17, 18) not covered by the screen (12) during the course of a measuring cycle, an indication of the direction and the extent of the displacement to which the support (7) of the object (8) to be examined is to be subjected so that during a measuring cycle no part of said object is placed between the source of radiation (4) and the detectors (17, 18) not covered by the screen (12) and disposed at the ends of the line of detectors (5) ; and

— performing a measuring cycle in which the apparatus is disposed in this way in the centred position.

2. A fan-beam tomodensitometer substantially comprising a source (4) emitting a fan-shaped beam of rays towards a plurality of detectors (5) arrayed in a line and defining a sector of the cutting plane in the interior of which there is provided a support (7) for the object (8) to be examined, in which the source of radiation (4) and the detectors (5) are mounted on a support (3) rotatable by a rotating means (2) attached to a frame (1) about an axis perpendicular to the cutting plane, a computer (19) which receives the signals emitted by each of the detectors (5) in any position of the rotatable support (3) during rotation thereof, a control console (21) mainly for controlling the computer (19), and a device (22) for evaluating the results outputted by the computer (19) as a function of the signals received from the detectors (5) and the programme received from the control console (21), characterised in that it further comprises a screen (12) which is opaque and movable between a centred position (12) and a retracted position (16) and in its centred position is disposed between the source of radiation (4) and the object (8) to be examined and which is dimensioned such that in the centred position (12) thereof it will keep off the rays striking the detectors (5) except for at least one detector (17, 18) disposed at at least one end of the line of detectors (5).

3. A tomodensitometer according to claim 2, characterised in that the opaque screen (12) is attached to means (13) adapted to receive opaque screens of different dimensions.

4. A tomodensitometer according to claim 2, characterised in that the opaque screen (12) is attached to means (13) which on command may be moved either towards the source of radiation (4) or opposite thereto.

5. A tomodensitometer according to claim 2, characterised in that the support (7) for the object (8) to be examined is attached to the frame (1) through means (9) which upon command may effect movement of the support in any desired direction.

**Ansprüche**

1. Verfahren zur automatischen Zentrierung eines zu untersuchenden Gegenstandes (8) in einem Fächerbündel-Tomodensitometer, wobei das Tomodensitometer im wesentlichen aus folgenden Bestandteilen besteht : einer Quelle (4), die ein Strahlenbündel in Fächerform in Richtung auf mehrere in einer Reihe angeordnete Detektoren (5) aussendet und einen Sektor der Schnittebene definiert, in dessen Inneren sich eine Halterung (7) für den zu untersuchenden Gegenstand befindet, wobei Quelle und Detektoren auf einer Halterung (3) montiert sind, die mit Hilfe einer auf einem Rahmen (1) befestigten Drehvorrichtung (2) um eine zur Schnittebene senkrechte Achse drehbar ist, und die Halterung (7) für den zu untersuchenden Gegenstand am Rahmen mit einer Vorrichtung (9) befestigt ist, die es erlaubt, daß die Halterung in eine beliebige Richtung verschiebbar ist ; einem Rechner (19), der die durch jeden Detektor (5) in jeder Stellung der drehbaren Halterung (3) im Verlauf ihrer Drehung ausgesandten Signale empfängt, einem Pult (21) zur Steuerung namentlich des Rechners (19) sowie einem Gerät (22) zur Auswertung der Ergebnisse, die von dem Rechner (19) als Funktion der von den Detektoren (5) erhaltenen Signale sowie des von dem Steuerpult (21) erhaltenen Programms ausgegeben werden ; und das Verfahren dadurch gekennzeichnet ist, daß man

— zwischen die Quelle (4) und dem zu untersuchenden Gegenstand (8) einen Schirm (12) anordnet, der für die Strahlen undurchlässig und zwischen einer zentrierten Stellung (12) und einer zurückgezogenen Stellung (16) bewegbar ist sowie solche Abmessungen aufweist, daß er in der zentrierten Stellung die Strahlen abhält, die auf die Detektoren (5) mit Ausnahme von mindestens einem Detektor (17, 18) an mindestens einem Ende der Reihe der Detektoren (5) auftreffen ;

— in den Rechner (19) ein Programm eingibt, um als Antwort auf die Signale, die der Rechner von dem Detektor oder den Detektoren (17, 18), die von dem Schirm (12) nicht abgedeckt werden, im Verlauf eines Meßzyklus erhält, die Angabe der Richtung und des Ausmasses der Verschiebung zu erhalten, der die Halterung (7) des zu untersuchenden Gegenstandes (8) zu unterziehen ist, damit nicht ein Teil des Gegenstandes während eines Meßzyklus zwischen die Strahlungsquelle (4) und die nicht von dem Schirm (12) abgedeckten Detektoren (17, 18), die sich an den Enden der Reihe der Detektoren (5) befinden, zu liegen kommt ; und

— einen Meßzyklus durchführt, wobei der Apparat auf diese Weise in der zentrierten Stellung angeordnet ist.

2. Fächerbündel-Tomodensitometer, im wesentlichen bestehend aus einer Quelle (4), die ein Strahlenbündel in Fächerform in Richtung auf mehrere Detektoren (5) aussendet, die in einer Reihe angeordnet sind und einen Sektor der Schnittebene definieren, in dessen Innerem sich eine Halterung (7) für den zu untersuchenden Gegenstand (8) befindet, wobei die Strahlungsquelle (4) und die Detektoren (5) auf eine Halterung (3) montiert sind, die mit Hilfe einer auf einem Rahmen (1) befestigten Drehvorrichtung (2) um eine senkrecht zur Schnittebene stehenden Achse drehbar ist, einem Rechner (19), der die von jedem Detektor (5) bei jeder Stellung der drehbaren Halterung (3) während ihrer Rotation ausgesandten Signale empfängt, einem Pult zur Steuerung (21) namentlich des Rechners (19) sowie einem Gerät (22) zur Auswertung der von dem Rechner (19) als Funktion der von den Detektoren (5) empfangenen Signale sowie des von dem Steuerpult (22) empfangenen Programms ausgegebenen Ergebnisse, dadurch gekennzeichent, daß es außerdem einen Schirm (12) enthält, der strahlungsundurchlässig und zwischen einer zentrierten Stellung (12) sowie einer zurückgezogenen Stellung (16) verschiebbar sowie in zentrierter Stellung zwischen der Strahlungsquelle (4) und dem zu untersuchenden Gegenstand (8) angeordnet ist und der derartige Abmessungen aufweist, daß er in der zentrierten Stellung (12) die Strahlen abhält, die auf die Detektoren mit Ausnahme mindestens eines Detektors (17, 18), der an mindestens einem der Enden der Reihe der Detektoren (5) angeordnet ist, auftreffen.

3. Tomodensitometer gemäß Anspruch 2, dadurch gekennzeichnet, daß der strahlungsundurchlässige Schirm (12) an einer Vorrichtung (13) befestigt ist, die strahlungsundurchlässige Schirme mit verschiedenen Abmessungen aufnehmen kann.

4. Tomodensitometer gemäß Anspruch 2, dadurch gekennzeichnet, daß der strahlungsundurchlässige Schirm (12) an einer Vorrichtung (13) befestigt ist, die sich auf Befehl in Richtung der Strahlungsquelle (4) oder in entgegengesetzter Richtung verschieben läßt.

5. Tomodensitometer gemäß Anspruch 2, dadurch gekennzeichnet, daß die Halterung (7) des zu untersuchenden Gegenstandes (8) am Rahmen (1) mittels einer Vorrichtung (9) befestigt ist, die auf Befehl eine Verschiebung der Halterung in beliebige Richtung bewirken kann.